(19) Europäisches Patentamt / European Patent Office / Office européen des brevets

(11) **EP 1 310 460 B1**

(12) **FASCICULE DE BREVET EUROPEEN**

(45) Date de publication et mention
de la délivrance du brevet:
**10.10.2012 Bulletin 2012/41**

(51) Int Cl.:
*C02F 1/36* (2006.01)          *C02F 1/72* (2006.01)
*C02F 1/32* (2006.01)          *A61L 2/02* (2006.01)
*A61L 2/00* (2006.01)          *A61L 2/025* (2006.01)
*B01J 19/00* (2006.01)         *B01J 19/10* (2006.01)

(21) Numéro de dépôt: **03075372.7**

(22) Date de dépôt: **04.07.1997**

(54) **DISPOSITIF ÉMETTEUR D'ULTRASONS ET MICROBULLES DE GAZ ET PROCÉDÉ DE TRAITEMENT DE CELLULES ET MICROORGANISMES**

VORRICHTUNG ENTHALTEND ULTRASCHALLESENDER UND GASMIKROBLASEN UND VERFAHREN ZUR BEHANDLUNG VON ZELLEN UND MIKROORGANISMEN.

ULTRASON AND GASMICROBUBBLES EMITTING DEVICE AND PROCESS FOR TREATING CELLULES AND MICROORGANISMS

(84) Etats contractants désignés:
**AT BE CH DE DK ES FI FR GB GR IE IT LI LU NL PT SE**

(30) Priorité: **04.07.1996 BE 9600613**

(43) Date de publication de la demande:
**14.05.2003 Bulletin 2003/20**

(62) Numéro(s) de document de la (des) demande(s) initiale(s) en application de l'article 76 CBE:
**97929045.9 / 0 912 445**

(73) Titulaire: **Ashland Licensing and Intellectual Property LLC**
**Dublin, OH 43017 (US)**

(72) Inventeurs:
• **Cordemans De Meulenaer, Eric**
**1970 Wezembeek-Oppem (BE)**
• **Hannecart, Baudouin**
**1180 Uccle (BE)**
• **Canivet, Yves**
**1140 Evere (BE)**

(74) Mandataire: **Van Malderen, Joëlle et al**
**pronovem - Office Van Malderen**
**Avenue Josse Goffin 158**
**1082 Bruxelles (BE)**

(56) Documents cités:
**EP-A- 0 619 104          EP-A- 0 661 090**
**EP-A- 0 680 779          GB-A- 1 389 291**

• **HENGLEIN A, HERBURGER D, GUTTIEREZ M: "Sonochemistry: Some Factors That Determine the Ability of a Liquid to Cavitate in an Ultrasonic Field" J. PHYS. CHEM., vol. 96, no. 3, 1992, pages 1126-1130, XP002345663**

## Description

### Objet de l'invention

La présente invention concerne un nouveau dispositif et un procédé de traitement d'un milieu liquide destinés à éliminer des micro-organismes tels que des algues, des bactéries, des virus, etc., et/ou destinés à permettre une sursaturation en sels dans le milieu liquide.

### Arrière-plan technologique

Il est connu de l'homme de l'art que selon que l'on utilise des ultrasons de hautes fréquences ou de basses fréquences, on obtient sur des fluides des effets différents. Pour obtenir des effets mécaniques, les basses fréquences (généralement comprises entre 16 et 100 kHz) sont régulièrement utilisées.

Ces effets mécaniques qui sont dus à la cavitation, permettent par exemple de mélanger, d'émulsifier et de disperser des substances hétérogènes, de dégazer des liquides, etc. Ces effets mécaniques créent également de forts effets de cisaillement au sein des liquides, ce qui permet par exemple de perturber les cellules vivantes présentes dans ce milieu et provoquer jusqu'à la rupture des parois de ce type de cellules.

Ces mêmes effets mécaniques sont également utilisés pour nettoyer des surfaces par l'action des microjets de liquide produits par l'implosion des bulles de cavitation. De même, le fait de pouvoir mélanger très efficacement divers réactifs est utilisé de manière avantageuse pour favoriser les phénomènes de diffusion dans diverses réactions chimiques, surtout les réactions hétérogènes.

Si l'on utilise des hautes fréquences, généralement comprises entre 300 kHz et plusieurs MHz, les phénomènes de cavitation deviennent relativement moins énergétiques, et les effets mécaniques sont plus réduits que lorsque l'on utilise des basses fréquences.

En appliquant des ultrasons de hautes fréquences à un milieu liquide, la durée de vie des bulles de cavitation produites est fortement réduite, et l'on assiste à des phénomènes chimiques en phase homogène qui se distinguent radicalement de ceux observés avec l'application des fréquences plus basses.

Un paramètre propre à l'utilisation d'ultrasons de hautes fréquences est que malgré la faible énergie mécanique nécessaire pour obtenir un dégazage des solutions, l'effet obtenu est plus élevé que lorsque l'on applique des ultrasons de basses fréquences qui, eux, nécessitent plus de puissance.

### Etat de la technique

Il est connu de différents documents de l'état de la technique que l'emploi l'ultrasons permet la détoxication de milieux liquides.

Le brevet américain US-5198122 décrit un procédé de détoxication de matériaux liquides et solides comprenant différents composés organiques toxiques, consistant en l'adjonction au milieu d'un catalyseur comprenant un borohydrate de sodium ou un hydrate de lithium aluminium aux dits matériaux, qui sont ensuite soumis aux ultrasons de manière à provoquer un phénomène de cavitation dans le mélange et à permettre la détoxication ou l'extraction plus aisée des substances contaminantes.

La demande de brevet allemand DE-4407564 décrit un procédé d'oxydation de substances organiques ou de micro-organismes obtenu par addition d'oxydants (ozone) ou d'agents catalyseur de transfert de phase.

La demande de brevet japonais JP-930343777 (Marsima Aqua Syst. Corp.) décrit un procédé et une installation de purification d'un réservoir d'eau contenant du plancton végétal. Dans le premier réservoir de cette installation, on inactive le plancton végétal au moyen d'un vibrateur à ultrasons provoquant des phénomènes de cavitation dans l'eau. Le produit de ce traitement est ensuite transféré dans un second réservoir, où l'on favorise la prédation du plancton animal sur le plancton végétal inactivé.

De même, il a été proposé dans différents documents (JP-900401407 (Ina Shokuhin Kogyo KK), JP-920035473 (Kubota Corp.), JP-920035472 (Kubota Corp.) et JP-920035896 (Kubota Corp.) de provoquer l'élimination de substances organiques chlorées ou d'éliminer des micro-organismes par un phénomène de cavitation au moyen d'ultrasons et d'injections d'ozone, de peroxydes et/ou de catalyseurs.

La demande de brevet japonais JP-820010627 (Hitachi Kiden Kogyo KK) décrit un procédé d'élimination de spores d'algues par un effet mécanique provoqué par l'utilisation d'ultrasons de hautes et basses fréquences, induisant la stérilisation et l'élimination des spores venant se fixer sur des plaques, et qui seront ensuite éliminés par l'effet purificateur de la cavitation des ultrasons de basses fréquences.

Tous ces exemples précédents utilisent les effets mécaniques des ultrasons de basses fréquences.

Il est connu par la publication scientifique de Pétrier C. et al. (Journal of Physical Chemistry, No. 98 - 10514-10520 (1994)) que la dégradation sonochimique des phénols dans des solutions aqueuses varie en fonction de la fréquence d'émission des ultrasons. Selon ce document, le taux de dégradation sonochimique serait directement lié à la présence dans la solution de radicaux libres comme par exemple H·, ·OH et HOO·, provenant de la sonolyse de l'eau sous ultrasons. Selon ce document, la production de ces radicaux libres, en particulier les radicaux ·OH et ·OOH, serait plus importante en travaillant avec l'émission d'ultrasons de hautes fréquences.

Comme il apparaît dans les illustrations de ce document, les radicaux produits par ultrasons induisent une fluorescence dont la distribution varie en fonction de la conception et du dessin de l'appareillage ainsi que de la fréquence d'émission des ultrasons. Cette fluorescence induite par les radicaux produits sous ultrasons est mise en évidence par l'adjonction de luminol à la composition aqueuse.

Cependant, ce document ne suggère nullement que l'on pourrait utiliser le phénomène d'oxydation obtenu par des ultrasons de hautes fréquences pour attaquer des micro-organismes présents dans ce milieu aqueux et obtenir ainsi leur élimination.

Le brevet américain US-2717874 décrit un dispositif de traitement d'un milieu aqueux comprenant des micro-organismes, par l'adjonction de réactifs chlorés. Ledit dispositif comprend une cloche (cellule) communiquant avec un réservoir du milieu aqueux à traiter, et comporte un émetteur d'ultrasons de hautes fréquences situé à la base de la cloche. En outre, la cloche présente un dispositif émetteur de bulles d'air au niveau du cône du geyser.

La demande de brevet WO93/13674 décrit un procédé de pasteurisation de lait ou de produits lactés de manière à éliminer un certain nombre de micro-organismes tels que des bactéries, des virus, des spores, etc. Le dispositif et le procédé décrits dans ce document sont basés sur l'utilisation de la cavitation obtenue à basses fréquences et à haute puissance pendant des temps d'application particulièrement longs.

La demande de brevet européen EP-A1-0633049 décrit un procédé de traitement d'un liquide par l'émission d'un champ d'ultrasons de manière à séparer les particules présentes dans ce liquide. Ce procédé utilise des hautes fréquences, qui permettent la séparation des microparticules par des champs d'ondes stationnaires dont on modifie la position par une variation de phase ou de fréquence, qui entraîne les particules qui restent ainsi concentrées dans les zones de ventres ou de noeuds. Dans le cas de particules biologiques, ce dispositif permet de créer un bioréacteur où les particules solides de toute nature restent confinées dans le champ ultrasonore, ce qui permet une filtration du milieu liquide. Il est à signaler que le dispositif permet d'obtenir la stérilisation de matériaux biologiques et l'inactivation de micro-organismes.

Le brevet américain US-4961860 décrit un procédé de traitement de l'eau, de manière à éliminer les micro-organismes présents par l'émission d'ultrasons de puissance à basses fréquences. Cependant, ce dispositif requiert une consommation énergétique particulièrement importante, de manière à obtenir un effet mécanique éliminant les micro-organismes.

EP-A-0 619 104 décrit un apparatus et un procédé de traitement de moyens liquides en utilisant un générateur d'ondes ultrasonores. GB-A-1 389 291 décrit un procédé et apparatus pour le traitement des déchets utilisant de l'énergie acoustique.

## Buts de l'invention

La présente invention vise à fournir un dispositif et un procédé susceptibles de neutraliser, d'empêcher le développement et/ou d'éliminer de manière simple et peu coûteuse un certain nombre de cellules animales non différenciées telles que des cellules tumorales et/ou des micro-organismes d'un milieu liquide, en particulier des algues, des bactéries, des virus, etc., et qui ne présenteraient pas les inconvénients de l'état de la technique.

Un but particulier de la présente invention est de neutraliser, d'empêcher le développement et/ou d'éliminer lesdites cellules non différenciées ou micro-organismes, en particulier des algues, présents dans des milieux liquides tels que les eaux de piscine, les châteaux d'eau, les viviers, les aquariums ou tout autre réservoir d'eau, et par extension tout circuit industriel, par exemple un circuit de refroidissement, dont la qualité de l'eau est particulièrement importante, ou des liquides physiologiques tels que du sang ou du plasma, pouvant être extraits et/ou administrés à l'homme ou l'animal.

L'invention vise également à obtenir un dispositif et un procédé qui ne nécessitent pas l'adjonction de produits chimiques additionnels nécessaires pour obtenir les effets susmentionnés dans le milieu traité.

Un autre but de l'invention est d'obtenir un dispositif et un procédé ne consommant pas beaucoup d'énergie.

Un but complémentaire de la présente invention est d'obtenir un dispositif et un procédé permettant de traiter un milieu liquide avec des ultrasons n'engendrant pas de phénomène stationnaire.

Un dernier but de la présente invention est de fournir un dispositif et un procédé de traitement d'un milieu aqueux, permettant de provoquer une sursaturation en sels du milieu liquide, c'est-à-dire permettant d'obtenir des concentrations en sels supérieures à la solubilité naturelle de ces sels dans le milieu aqueux.

## Eléments caractéristiques de l'invention

L'objet de la présente demande est défini dans les libellés des revendications indépendantes 1, 15 et 19. D'autres aspects de la demande sont décrits dans les libellés des revendications dépendantes.

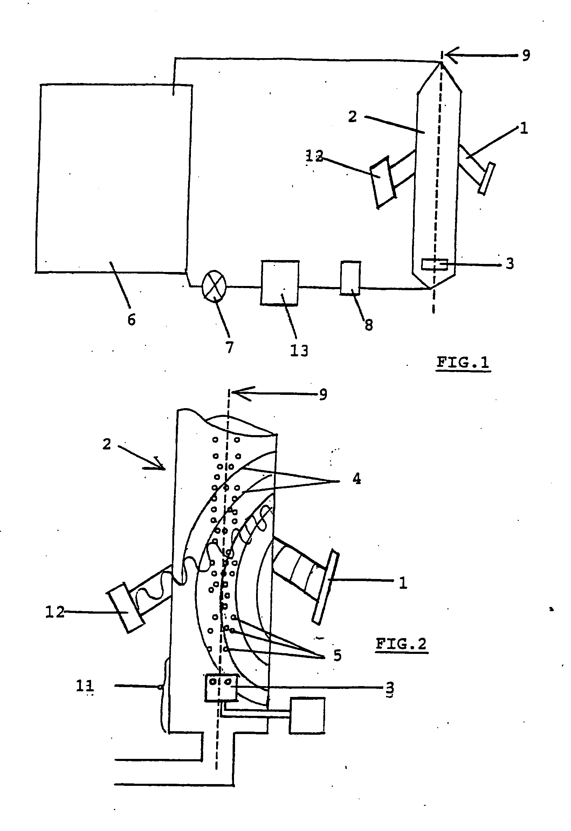

La présente invention telle que représentée dans les figures 1 et 2 concerne un dispositif ou installation de traitement d'un milieu liquide, de préférence aqueux (contenant éventuellement des cellules non différenciées ou des micro-organismes), caractérisé en ce qu'il comprend, communiquant de préférence avec un "réservoir" 6 du milieu liquide à traiter, un compartiment 2, de préférence de forme cylindrique ou à section carrée, ledit compartiment 2 comportant (le long de sa paroi) un ou plusieurs émetteurs 1 d'ultrasons de hautes fréquences 4 émis vers l'intérieur du compartiment 2 (de préférence vers le centre de ce compartiment 2), et un ou plusieurs émetteurs 3 de microbulles de gaz 5 disposés de manière à assurer l'émission des microbulles de gaz 5 dans le champ des ultrasons 4 émis dans le compartiment 2.

On entend par "microbulles", des bulles de gaz ayant un diamètre moyen inférieur à 1 mm, de préférence inférieur ou égal à 50 $\mu$m, plus particulièrement inférieur à 30 $\mu$m.

On entend par "cellules non différenciées", des cellules présentant un effet hyperprolifératif telles que des cellules tumorales, des cellules de moelle osseuse ou des cellules totipotentes, notamment des cellules d'algues, etc.

On entend par "micro-organismes" présents dans un milieu liquide que l'on désire traiter, tous micro-organismes pathogènes ou non pathogènes susceptibles de provoquer des désagréments à l'homme, l'animal ou aux installations de transport et de stockage de milieux liquides. De tels micro-organismes peuvent être des bactéries, des virus ou des algues. La définition de ces micro-organismes comprend des êtres uni- et pluricellulaires, en particulier des algues, pouvant être présents dans différents milieux liquides, de préférence aqueux tels que des piscines, des réservoirs d'eau, des aquariums, des circuits industriels de refroidissement telles que des tours de refroidissement, etc.

On entend également par "milieu liquide " des liquides physiologiques pouvant être administrés à l'homme ou l'animal, ou extraits de l'homme ou de l'animal et réinjectés à celui-ci après traitement (traitement ex vivo de liquides physiologiques tels que le sang, le sérum, le liquide céphalo-rachidien, etc.), comme décrit dans le brevet américain US-5401237.

On entend par "hautes fréquences", des fréquences comprises entre 100 kHz et plusieurs MHz. De préférence, les hautes fréquences utilisées sont comprises entre 200 kHz et 10 MHz.

De préférence, ledit émetteur 3 de microbulles de gaz 5 est disposé à la base 11 du compartiment 2, c'est-à-dire dans la partie inférieure du compartiment 2, de manière à ce que les microbulles se déplacent par remontée naturelle ou entraînement du gaz dans le flux de liquide.

Les microbulles de gaz sont des microbulles d'air ou d'oxygène.

Selon l'invention, lesdits micro-organismes semblent être éliminés par la production dans leur environnement de radicaux de type H·, ·OH et HOO· susceptibles également de former de l'$H_2O_2$, cette molécule et/ou ces radicaux étant toxiques pour ces micro-organismes et provoquant ainsi leur inactivation et/ou leur destruction.

Les espèces créées proviendraient des réactions des ultrasons de hautes fréquences sur la molécule d'eau qui pourraient provoquer (en particulier en présence d'oxygène) les réactions suivantes :

$$H_2O \dashrightarrow H\cdot + \cdot OH$$

$$H\cdot + O_2 \dashrightarrow HOO\cdot$$

$$HOO\cdot + HOO\cdot \dashrightarrow H_2O_2 + O_2$$

$$\cdot OH + \cdot OH \dashrightarrow H_2O_2$$

De manière avantageuse, l'énergie nécessaire pour produire les espèces toxiques est réduite si l'on travaille en présence de ces microbulles.

En effet, il semblerait que l'injection de microbulles dans le champ des ultrasons provoque une augmentation du phénomène de sonoluminescence, par la superposition des microbulles aux bulles de cavitation provoquées par les ultrasons, ce qui semble multiplier le nombre d'espèces excitées et toxiques.

Ce phénomène est massivement observé lorsque l'on combine de manière synergique le traitement des ultrasons en présence des microbulles d'une taille adéquate.

Ce phénomène est accentué par la fragmentation des microbulles 5 créées lorsqu'elles passent dans le champ des ultrasons 4, en particulier lorsque le dispositif comporte plusieurs émetteurs successifs le long de la paroi du compartiment 2, qui assurent une plus grande fragmentation des microbulles et ce, de manière progressive.

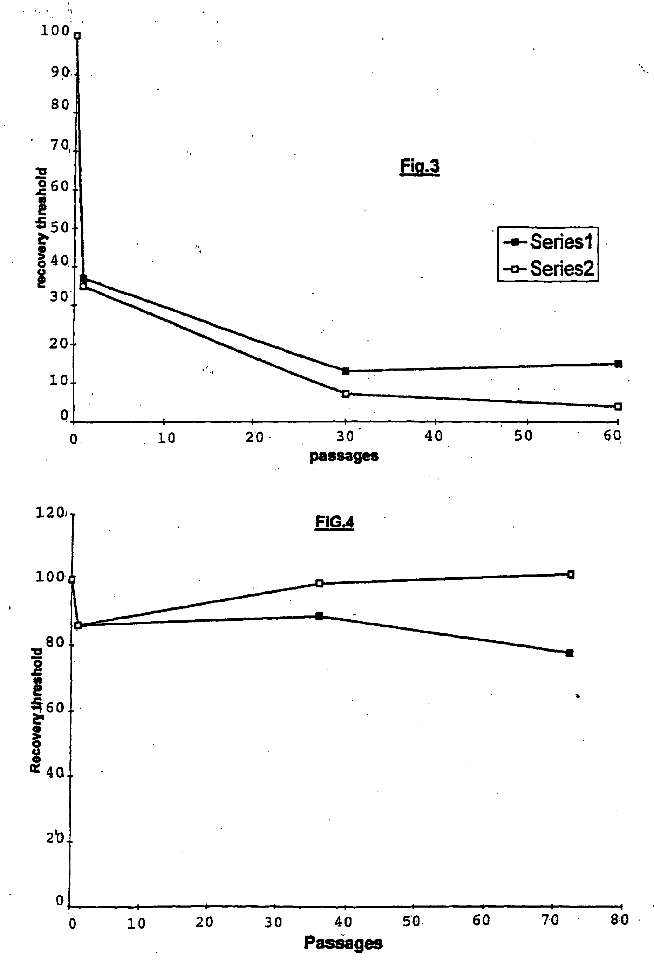

Ces caractéristiques sont notamment illustrées par les tests comparatifs des figures 3 à 5 annexées.

Les figures 3 et 4 reprennent un comptage des algues de type selenastrum capricornutum. On observe que ces algues placées dans des conditions optimales de culture voient leur potentiel de récupération (recovery threshold) inhibé lorsqu'elles sont traitées par le dispositif de l'invention (avec injection de microbulles (figure 3) et sans injection de microbulles (figure 4)). Pour une puissance ultrasonore donnée, l'efficacité du traitement est proportionnelle au temps de séjour dans le compartiment, et par conséquent, inversement proportionnelle au débit.

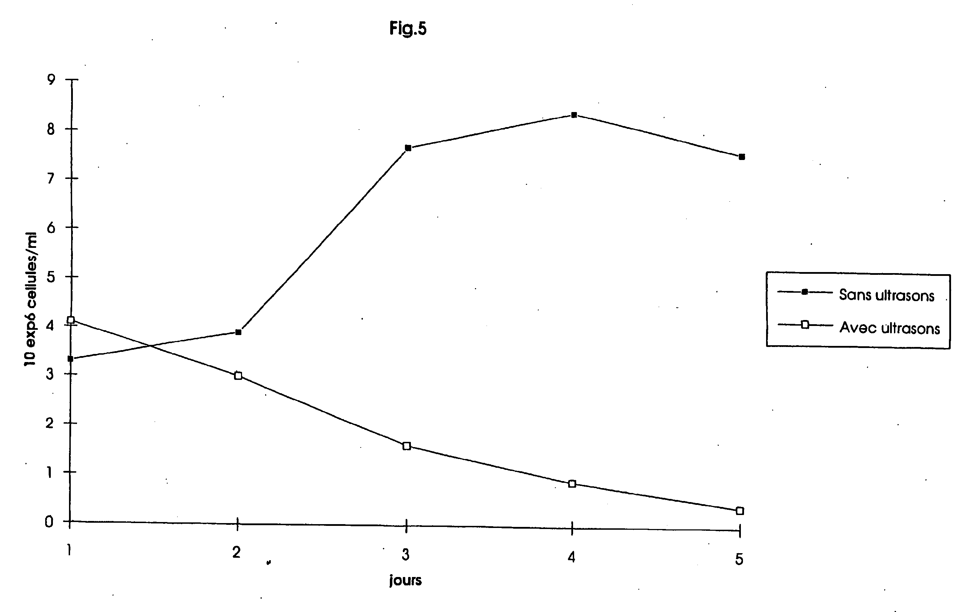

La figure 5 démontre, par rapport à un blanc de référence (traité dans toutes conditions de concentration, débit, température et bullage égales mais sans ultrasons), l'effet des ultrasons. On observe qu'un traitement par ultrasons mais sans injection de microbulles ne provoque pas d'inhibition de la croissance ni de diminution du nombre d'algues dans le milieu liquide.

En outre, le dispositif présente l'avantage qu'il n'y a pas lieu de dédicacer les ultrasons dans des zones particulières, car on constate que le système de traitement fonctionne par diffusion des produits formés in situ (par exemple radicaux et $H_2O_2$ formés) vers le réservoir 6 de milieu aqueux à traiter.

En outre, l'émetteur 1 d'ultrasons 4 du dispositif de l'invention est orienté de manière à ne pas provoquer de phénomènes d'ondes stationnaires, c'est-à-dire qu'il est orienté en oblique par rapport à l'axe 9 du compartiment 2 (angle aigu non perpendiculaire par rapport à cet axe 9) et par rapport au flux de liquide et au débit de microbulles 5 (voir figure 2). Cette caractéristique permet que toutes les microbulles 5 du compartiment 2 soient traitées de manière statistiquement identique, sans créer de zones stationnaires dans ledit compartiment 2.

Selon une forme d'exécution préférée de l'invention, le dispositif comprend également un émetteur de lumière 12 vers l'intérieur du compartiment 2 dans le champ des ultrasons 4, c'est-à-dire un émetteur de rayonnement électromagnétique dont la majorité de la fréquence est dans le visible. Cependant, pour certaines applications, de manière à éliminer certains micro-organismes particuliers, il peut être avantageux d'émettre également un rayonnement électromagnétique dont certaines fréquences correspondent au rayonnement ultraviolet (de type UVA, UVB ou UVC) ou d'autres types d'émissions énergétiques tels que infrarouge, laser, micro-ondes, etc.

En effet, les Inventeurs ont observé de manière inattendue qu'un traitement comprenant l'émission de microbulles dans les champs combinés des ultrasons et du rayonnement lumineux, était particulièrement efficace pour obtenir une inactivation et une élimination des micro-organismes, en particulier des algues, présents dans le milieu liquide. Le phénomène de sonoluminescence peut favoriser la production d'espèces oxygénées extrêmement actives telles que le radical superoxyde ou l'oxygène de type singulet, qui sont responsables de toute une série de réactions biochimiques, extrêmement toxiques pour certains micro-organismes.

Il est connu qu'un tel phénomène peut être produit en présence de molécules dites photosensibilisatrices de manière à provoquer une action anti-tumorale sur certaines cellules cancéreuses (Umemura S.I., Japanese J. of Cancer Res., 81, pp. 962-966 (1990)). De telles molécules sont notamment des porphyrines, des chlorines, des tétracyclines, du bleu de méthylène, de la fluorescéine, de l'acridine, de la rhodamine, etc. Ces agents actifs sont injectés dans l'organisme ou administrés par voie orale pour être ensuite activés par sonoluminescence et produire ainsi l'oxygène de type singulet qui à son tour joue un rôle capital, notamment dans les processus biochimiques dus au stress oxydatif. L'action de l'oxygène singulet est d'oxyder les divers composants des cellules que sont les protéines, les lipides, les acides aminés et les nucléotides.

Selon la présente invention, il n'est pas toujours nécessaire d'ajouter dans le milieu à traiter un agent photosensibilisateur. En effet, les Inventeurs ont observé de manière inattendue que cet effet pouvait être produit in situ sur certains micro-organismes, notamment des algues, ou certaines cellules, en particulier des cellules non différenciées telles que des cellules tumorales présentes dans des liquides physiologiques tels que le sang, contenant déjà ces molécules photosensibilisatrices.

Dans le cas où l'on traite un liquide physiologique humain ou animal, le dispositif de la présente invention peut ne pas comporter de réservoir 6 et être directement relié au corps humain ou animal duquel le liquide physiologique est prélevé et réinjecté par un procédé de traitement extra-corporel du corps humain ou animal.

Selon une forme d'exécution préférée de l'invention, telle que représentée à la figure 1, le dispositif comprend, placé entre le réservoir 6 et le compartiment 2, un moyen de traitement électromagnétique 13 du milieu aqueux. Ce moyen 13 est donc séparé du compartiment 2 et placé en amont de celui-ci par rapport au flux du milieu aqueux à traiter.

On entend par "moyen de traitement électromagnétique d'un milieu aqueux", tout appareillage permettant de modifier, voire orienter, la formation de cristaux (modification de la morphologie des cristaux).

Un tel appareillage est notamment décrit dans la demande de brevet européen EP-0515346 qui décrit un générateur permettant de contrôler la nucléation et/ou la croissance de cristaux et/ou leur morphologie. Ce document décrit un exemple particulier permettant d'orienter les cristaux de carbonate de calcium vers une forme dendritique, ces cristaux étant dans ce cas dans une forme inapte à former des incrustations de tartre (dans les canalisations, échangeurs, etc.).

Un tel moyen de traitement électromagnétique du milieu aqueux permet, lorsque l'on soumet ledit milieu aqueux à l'action d'un champ créé entre au moins deux moyens de transfert (de manière à modifier la nucléation et/ou la formation et/ou la croissance de cristaux, en particulier de carbonate de calcium), au moins un desdits moyens de transfert recevant des signaux électriques sensiblement rectangulaires comprenant une première phase dans laquelle le signal est voisin d'un niveau inférieur et une deuxième phase dans laquelle le signal est voisin d'un niveau supérieur, lesdits signaux présentant, lorsqu'ils sont voisins du niveau inférieur ou du niveau supérieur, une série de variations amorties.

De façon avantageuse, les signaux correspondent à la superposition d'une onde rectangulaire de fréquence inférieure à 100000 Hz, de préférence comprise entre 1000 et 30000 Hz, et d'une onde sinusoïdale amortie de fréquence inférieure à 100000 Hz, de préférence comprise entre 1 MHz et 15 MHz, et l'amortissement de l'onde sinusoïdale est telle qu'après 5 périodes de ladite onde, l'amplitude de la variation est inférieure à 10% de celle de la variation initiale.

Dans une forme d'exécution particulière de l'invention, pour chaque phase de l'onde rectangulaire, cette dernière est associée à une seule onde sinusoïdale amortie, la fréquence de l'onde amortie étant supérieure à celle de l'onde rectangulaire et l'onde amortie étant émise pour chaque phase de l'onde rectangulaire, le temps de vie de l'onde amortie étant compris entre 10 ns et 500 μs, de préférence entre 100 ns ET 100 μs.

Dans un exemple de réalisation particulier, la différence de potentiel existant entre le niveau inférieur et le niveau supérieur de l'onde rectangulaire est comprise entre 5 et 200 V, de préférence entre 12 et 60 V, tandis que les ondes amorties sont des ondes dont les variations sont situées dans une bande s'étendant entre un niveau minimum et un niveau maximum, la différence de potentiel entre lesdits niveaux étant comprise entre 1 et 60 V, de préférence entre 10 et 35 V.

Ledit moyen de traitement électromagnétique d'un milieu aqueux peut être également l'appareillage tel que décrit dans le brevet américain US-4879045.

Les Inventeurs ont découvert de manière inattendue que le dispositif de l'invention, comprenant un moyen assurant un traitement électromagnétique du milieu liquide disposé en amont des éléments assurant un traitement du milieu aqueux par microbullage dans un champ d'ultrasons de hautes fréquences, permet d'obtenir une large plage de sursaturation en sels du milieu liquide avant que le phénomène de cristallisation n'apparaisse.

On entend par "sursaturation en sels", la possibilité d'augmenter la concentration en sels du milieu aqueux (au-delà de la concentration correspondant au produit de solubilité des sels) sans obtenir une précipitation desdits sels.

Par exemple, lesdits sels sont constitués par du carbonate de calcium présent dans le milieu aqueux, qui peut être apporté via un raccordement du milieu aqueux à de l'eau du réseau de captage et de distribution ou par ajout desdits sels dans le milieu liquide.

Cette quantité importante de sels présente dans le milieu aqueux peut également être obtenue par une évaporation partielle de l'eau, ce phénomène étant particulièrement important dans les tours de refroidissement.

Le milieu liquide peut également comprendre une sursaturation en d'autres sels, tels que des phosphates ou des sulfates de métaux alcalins ou alcalino-terreux, etc.

De manière inattendue, les Inventeurs ont observé qu'il est possible d'obtenir une concentration en sels, en particulier en carbonate de calcium, dans le milieu liquide traité, à des concentrations 10 fois supérieures à la saturation (dissolution maximale avant précipitation).

De manière avantageuse, on observe également que l'eau ainsi traitée reste limpide.

Un tel dispositif permet de développer des systèmes anti-tartre, de produire des eaux sursaturées en sels, de provoquer des effets biotoxiques sur certains micro-organismes dus à la présence d'eau sursaturée en certains sels, etc.

Un tel dispositif permet également d'obtenir des milieux concentrés sans précipitation et créer des réactions chimiques particulières par adjonction de réactifs.

De manière inattendue, les Inventeurs ont découvert que pour obtenir de manière optimale ce phénomène de sursaturation en sels des milieux aqueux traités, il est nécessaire que le dispositif de traitement électromagnétique soit séparé du compartiment où se fait l'émission de microbulles dans le champ d'ultrasons, et de préférence qu'il la précède.

Le dispositif selon la présente invention peut comprendre, disposés entre le réservoir 6 contenant le milieu liquide à traiter et le tube 2, une pompe 7 assurant la circulation du milieu liquide, ainsi que des moyens de récupération, de préférence par filtration, centrifugation ou précipitation (tels que des cyclones, etc.), des micro-organismes et/ou des sels présents dans le milieu liquide.

Un autre aspect de la présente invention concerne l'utilisation du dispositif selon l'invention pour neutraliser et/ou éliminer les micro-organismes, en particulier des algues, présents dans un milieu liquide à traiter.

On entend par "neutralisation et/ou élimination d'un micro-organisme", la réduction ou le maintien en-dessous d'un niveau de concentration (variant pour chaque micro-organisme et chaque milieu liquide) des micro-organismes susceptibles de se multiplier dans ce milieu liquide.

Un dernier aspect de la présente invention concerne l'utilisation du dispositif selon l'invention pour provoquer une sursaturation en sels du milieu liquide, de préférence du milieu aqueux, c'est-à-dire obtenir des concentrations en sels supérieures à la solubilité naturelle de ces sels dans le milieu liquide, de préférence une concentration 5 fois, voire 10 fois, supérieure à la solubilité naturelle de ces sels.

Les milieux sursaturés ainsi obtenus permettent leur utilisation pour provoquer des réactions chimiques particulières par adjonction de réactifs, conférant aux produits formés des caractéristiques originales (taille, forme, surface spécifique, etc.). Les réactifs ajoutés peuvent être solides, liquides, gazeux ou constitués de mélanges hétérogènes.

## Revendications

1. Dispositif de traitement d'un milieu liquide comprenant un compartiment (2) comportant un ou plusieurs émetteurs (1) d'ultrasons (4) émis vers l'intérieur du compartiment (2) et des émetteurs (3) de microbulles de gaz (5) présentant un diamètre moyen inférieur à 1 mm, **caractérisé en ce que** ledit émetteur (1) d'ultrasons (4) est un émetteur (1) d'ultrasons (4) de hautes fréquences comprises entre 100 KHz et plusieurs MHz, **en ce que** les émetteurs (3) de microbulles de gaz (5) sont disposés de manière à assurer l'émission des microbulles de gaz (5) dans le champ des ultrasons (4) de hautes fréquences émis dans le compartiment (2) et **en ce que** les microbulles de gaz (5) sont des microbulles d'air ou d'oxygène.

2. Dispositif selon la revendication 1, **caractérisé en ce que** le milieu liquide est un milieu liquide aqueux.

3. Dispositif selon la revendication 2, **caractérisé en ce que** le milieu liquide est un liquide physiologique pouvant être administré à l'homme ou à l'animal ou extrait de l'homme ou de l'animal.

4. Dispositif selon la revendication 3, **caractérisé en ce que** le liquide physiologique est choisi parmi le groupe constitué par le sang, le sérum ou le liquide céphalo-rachidien.

5. Dispositif selon l'une quelconque des revendications précédentes, **caractérisé en ce que** le diamètre moyen des microbulles de gaz (5) est inférieur à 50 μm.

6. Dispositif selon l'une quelconque des revendications précédentes, **caractérisé en ce que** le diamètre moyen des microbulles de gaz (5) est inférieur à 30 μm.

7. Dispositif selon l'une quelconque des revendications précédentes, **caractérisé en ce que** les émetteurs (3) de microbulles de gaz (5) sont disposés à la base (11) du compartiment (2) et **en ce que** les microbulles de gaz (5) se déplacent par remontée naturelle ou entraînement des microbulles de gaz (5) dans le milieu aqueux à traiter.

8. Dispositif selon l'une quelconque des revendications précédentes, **caractérisé en ce que** le compartiment (2) présente une forme cylindrique ou une forme à section carrée.

9. Dispositif selon l'une quelconque des revendications précédentes, **caractérisé en ce que** l'émetteur (1) d'ultrasons (4) est orienté en oblique par rapport à l'axe (9) dudit compartiment (2).

10. Dispositif selon l'une quelconque des revendications précédentes, **caractérisé en ce que** le compartiment (2) comporte également un émetteur (12) de lumière émise dans le champ des ultrasons (4) et dont le rayonnement électromagnétique est majoritairement dans le visible.

11. Dispositif selon l'une quelconque des revendications précédentes, **caractérisé en ce qu'**il comporte un émetteur de rayonnement électromagnétique dont certaines fréquences correspondent au rayonnement ultraviolet (UVA, UVB, UVC) ou un autre type d'émission énergétique.

12. Dispositif selon la revendication 11 dans lequel l'autre type d'émission énergétique est choisi parmi le groupe constitué par l'infrarouge, le rayonnement laser ou les micro-ondes.

13. Dispositif selon l'une quelconque des revendications précédentes, **caractérisé en ce qu'**il comporte un ou plusieurs moyens permettant de recueillir les micro-organismes présents dans le milieu liquide traité.

14. Dispositif selon la revendication 13, **caractérisé en ce que** les moyens permettant de recueillir les micro-organismes présents dans le milieu liquide traité sont un filtre ou un cyclone.

15. Utilisation du dispositif selon l'une quelconque des revendications précédentes, pour neutraliser, éliminer et/ou empêcher le développement de cellules non différenciées présents dans un milieu liquide.

16. Utilisation selon la revendication 15, **caractérisée en ce que** les cellules non différenciées sont des cellules présentant un effet hyperprolifératif.

17. Utilisation selon la revendication 16, **caractérisée en ce que** les cellules présentant un effet hyperprolifératif sont choisies parmi le groupe constitué par les cellules cancéreuses et les cellules de la moelle osseuse.

18. Utilisation selon la revendication 15, **caractérisée en ce que** les cellules non différenciées sont des cellules totipotentes.

19. Procédé pour neutraliser, éliminer et/ou empêcher le développement de cellules non-différenciées présents dans un milieu liquide dans lequel des microbulles de gaz (5) présentant un diamètre moyen inférieur à 1 mm et des ultrasons (4) sont émis à l'intérieur d'un compartiment (2) comportant le milieu liquide à traiter, dans lequel lesdites microbulles de gaz (5) sont émises dans le champ d'ultrasons (4) émis dans ledit compartiment (2) **caractérisé en ce que** lesdits ultrasons (4) sont des ultrasons de hautes fréquences comprises entre 100 KHz et plusieurs MHz et dans lequel les microbulles de gaz (5) sont des microbulles d'air ou d'oxygène.

20. Procédé selon la revendication 19, **caractérisé en ce que** le milieu liquide est un milieu liquide aqueux.

21. Procédé selon la revendication 20, **caractérisé en ce que** le milieu liquide est un liquide physiologique pouvant être administré à l'homme ou à l'animal ou extrait de l'homme ou de l'animal.

22. Procédé selon la revendication 21, **caractérisé en ce que** le liquide physiologique est choisi parmi le groupe constitué par le sang, le sérum ou le liquide céphalo-rachidien.

**23.** Procédé selon l'une quelconque des revendications précédentes 19 à 22, **caractérisé en ce que** le diamètre moyen des microbulles de gaz (5) est inférieur à 50 μm.

**24.** Procédé selon l'une quelconque des revendications précédentes 19 à 23, **caractérisé en ce que** le diamètre moyen des microbulles de gaz (5) est inférieur à 30 μm.

**25.** Procédé selon l'une quelconque des revendications précédentes 19 à 24, **caractérisé en ce que** les ultrasons (4) émis dans le compartiment (2) n'engendrent pas de phénomène de champ stationnaire.

**26.** Procédé selon l'une quelconque des revendications précédentes 19 à 25, **caractérisé en ce que** de la lumière dont le rayonnement électromagnétique est majoritairement dans le visible, est émise dans le champ des ultrasons (4).

**27.** Procédé selon l'une quelconque des revendications précédentes 19 à 26, **caractérisé en ce que** les cellules non différenciées sont des cellules présentant un effet hyperprolifératif.

**28.** Procédé selon la revendication 27, **caractérisé en ce que** les cellules présentant un effet hyperprolifératif sont choisies parmi le groupe constitué par les cellules cancéreuses et les cellules de la moelle osseuse.

**29.** Procédé selon la revendication 27, **caractérisé en ce que** les cellules présentant un effet hyperprolifératif sont des cellules totipotentes.

**Claims**

**1.** A device for treating a liquid medium comprising a compartment (2) including one or several emitters (1) of ultrasonic waves (4) transmitted towards the interior of the compartment (2) and emitters (3) of gas microbubbles (5) having an average diameter of less than 1 mm, **characterized in that** said emitter (1) of ultrasonic waves (4) is an emitter (1) of ultrasonic waves (4) of high frequency comprised between 100 kHz and several MHz, **in that** the emitters (3) of gas microbubbles (5) are positioned so as to ensure the transmission of gas microbubbles (5) in the field of the high frequency ultrasonic waves (4) emitted into the compartment (2) and **in that** the gas microbubbles (5) are air or oxygen microbubbles.

**2.** The device according to claim 1, **characterized in that** the liquid medium is an aqueous liquid medium.

**3.** The device according to claim 2, **characterized in that** the liquid medium is a physiological liquid which may be administered to humans or animals or extracted from humans or animals.

**4.** The device according to claim 3, **characterized in that** the physiological liquid is selected from the group formed by blood, serum or cerebrospinal liquid.

**5.** The device according to any of the preceding claims, **characterized in that** the average diameter of the gas microbubbles (5) is less than 50 μm.

**6.** The device according to any of the preceding claims, **characterized in that** the average diameter of the gas microbubbles (5) is less than 30 μm.

**7.** The device according to any of the preceding claims, **characterized in that** the emitters (3) of gas microbubbles (5) are positioned at the base (11) of the compartment (2) and **in that** the gas microbubbles (5) move by natural ascent or entrainment of gas microbubbles (5) in the aqueous medium to be treated.

**8.** The device according to any of the preceding claims, **characterized in that** the compartment (2) has a cylindrical shape or a shape with a square section.

**9.** The device according to any of the preceding claims, **characterized in that** the emitter (1) of ultrasonic waves (4) is obliquely oriented relatively to the axis (9) of said compartment (2).

**10.** The device according to any of the preceding claims, **characterized in that** the compartment (2) also includes an emitter (12) of light emitted into the field of ultrasonic waves (4) and the electromagnetic radiation of which is in majority in the visible range.

**11.** The device according to any of the preceding claims, **characterized in that** it includes a emitter of electromagnetic radiation, certain frequencies of which correspond to ultraviolet radiation (UVA, UVB, UVC) or to another type of energy emission.

**12.** The device according to claim 11, wherein the other type of energy emission is selected from the group formed by infrared, laser radiation or microwaves.

**13.** The device according to any of the preceding claims, **characterized in that** it includes one or several means with which microorganisms in the treated liquid medium may be collected.

**14.** The device according to claim 13, **characterized in that** the means with which the microorganisms in the treated liquid medium may be collected, are a filter or a cyclone.

15. The use of the device according to any of the preceding claims, for neutralizing, removing and/or preventing the development of non-differentiated cells present in the liquid medium.

16. The use according to claim 15, **characterized in that** the non-differentiated cells are cells having a hyperproliferative effect.

17. The use according to claim 16, **characterized in that** the cells having a hyperproliferative effect, are selected from the group formed by cancer cells and bone marrow cells.

18. The use according to claim 15, **characterized in that** the non-differentiated cells are totipotent cells.

19. A method for neutralizing, removing and/or preventing development of non-differentiated cells present in the liquid medium wherein gas microbubbles (5) having an average diameter of less than 1 mm and ultrasonic waves (4) are emitted towards the interior of a compartment (2) including the medium to be treated, wherein said gas microbubbles (5) are emitted into the field of ultrasonic waves (4) emitted in said compartment (2), **characterized in that** said ultrasonic waves (4) are ultrasonic waves of high frequencies comprised between 100 kHz and several MHz and wherein the gas microbubbles (5) are air or oxygen microbubbles.

20. The method according to claim 19, **characterized in that** the liquid medium is an aqueous liquid medium.

21. The method according to claim 20, **characterized in that** the liquid medium is a physiological medium which may be administered to humans or animals or extracted from humans or animals.

22. The method according to claim 21, **characterized in that** the physiological liquid is selected from the group formed by blood, serum or cerebrospinal liquid.

23. The method according to any of the preceding claims 19 to 22, **characterized in that** the average diameter of the gas microbubbles (5) is less than 50 μm.

24. The method according to any of the preceding claims 19 to 23, **characterized in that** the average diameter of the gas microbubbles (5) is less than 30 μm.

25. The method according to any of the preceding claims 19 to 24, **characterized in that** the ultrasonic waves (4) emitted into the compartment (2) do not generate any stationary field phenomenon.

26. The method according to any of the preceding claims 19 to 25, **characterized in that** light for which electromagnetic radiation is in majority in the visible range, is emitted into the field of ultrasonic waves (4).

27. The method according to any of the preceding claims 19 to 26, **characterized in that** the non-differentiated cells are cells having a hyperproliferative effect.

28. The method according to claim 27, **characterized in that** the cells having a hyperproliferative effect are selected from the group formed by cancer cells and bone marrow cells.

29. The method according to claim 27, **characterized in that** the cells having a hyperproliferative effect are totipotent cells.

**Patentansprüche**

1. Verarbeitungsvorrichtung eines flüssigen Mediums, die ein Fach (2) umfasst, das einen oder mehrere Sender (1) von Ultraschallwellen (4) aufweist, die in das Innere des Fachs (2) gesendet werden, und Sender (3) von Gasmikrokugeln (5), die einen durchschnittlichen Durchmesser von unter 1 mm aufweisen, **dadurch gekennzeichnet, dass** der Sender (1) von Ultraschallwellen (4) ein Sender (1) von Ultraschallwellen (4) mit hohen Frequenzen zwischen 100 KHz und mehreren MHz inklusive ist, dass die Sender (3) von Gasmikrokugeln (5) derart angeordnet sind, dass die Sendung der Gasmikrokugeln (5) in den Bereich der hochfrequenten Ultraschallwellen (4), die in das Fach (2) gesendet werden, gesichert ist, und dass die Gasmikrokugeln (5) Luft- oder Sauerstoff-Mikrokugeln sind.

2. Vorrichtung nach Anspruch 1, **dadurch gekennzeichnet, dass** das flüssige Medium ein wässriges flüssiges Medium ist.

3. Vorrichtung nach Anspruch 2, **dadurch gekennzeichnet, dass** das flüssige Medium eine physiologische Flüssigkeit ist, die dem Menschen oder dem Tier verabreichbar oder dem Menschen oder dem Tier entnehmbar ist.

4. Vorrichtung nach Anspruch 3, **dadurch gekennzeichnet, dass** die physiologische Flüssigkeit aus der Gruppe ausgewählt ist, die von dem Blut, dem Serum oder der Hirn- und Rückenmarksflüssigkeit gebildet wird.

5. Vorrichtung nach einem der vorangehenden Ansprüche, **dadurch gekennzeichnet, dass** der durchschnittliche Durchmesser der Gasmikrokugeln (5) kleiner als 50 μm ist.

6. Vorrichtung nach einem der vorangehenden Ansprüche, **dadurch gekennzeichnet, dass** der durchschnittliche Durchmesser der Gasmikrokugeln (5) kleiner als 30 μm ist.

7. Vorrichtung nach einem der vorangehenden Ansprüche, **dadurch gekennzeichnet, dass** die Sender (3) von Gasmikrokugeln (5) an der Basis (11) des Fachs (2) angeordnet sind und dass sich die Gasmikrokugeln (5) durch natürliches Aufsteigen oder Antrieb der Gasmikrokugeln (5) in dem zu behandelnden wässrigen Medium bewegen.

8. Vorrichtung nach einem der vorangehenden Ansprüche, **dadurch gekennzeichnet, dass** das Fach (2) eine zylindrische Form oder eine Form mit quadratischem Querschnitt aufweist.

9. Vorrichtung nach einem der vorangehenden Ansprüche, **dadurch gekennzeichnet, dass** der Sender (1) von Ultraschallwellen (4) im Verhältnis zur Achse (9) des Fachs (2) schräg ausgerichtet ist.

10. Vorrichtung nach einem der vorangehenden Ansprüche, **dadurch gekennzeichnet, dass** das Fach (2) ebenfalls einen Sender (12) von Licht aufweist, das in das Feld der Ultraschallwellen (4) gesendet wird und dessen elektromagnetische Strahlung sich mehrheitlich im Sichtbaren befindet.

11. Vorrichtung nach einem der vorangehenden Ansprüche, **dadurch gekennzeichnet, dass** sie einen Sender elektromagnetischer Strahlung aufweist, von der bestimmte Frequenzen der ultravioletten Strahlung (UVA, UVB, UVC) entsprechen oder einem anderen Typ energetischer Sendung.

12. Vorrichtung nach Anspruch 11, wobei der andere Typ energetischer Sendung aus der Gruppe ausgewählt ist, die vom Infrarot, der Laserstrahlung oder den Mikrowellen gebildet wird.

13. Vorrichtung nach einem der vorangehenden Ansprüche, **dadurch gekennzeichnet, dass** sie ein oder mehrere Mittel aufweist, die es erlauben, die in dem behandelten flüssigen Medium vorhandenen Mikroorganismen zu sammeln.

14. Vorrichtung nach Anspruch 13, **dadurch gekennzeichnet, dass** die Mittel, die es erlauben, die in dem behandelten flüssigen Medium vorhandenen Mikroorganismen zu sammeln, ein Filter oder ein Zyklon sind.

15. Verwendung der Vorrichtung nach einem der vorangehenden Ansprüche, um die Entwicklung von nicht differenzierten Zellen, die in einem flüssigen Medium vorhanden sind, zu neutralisieren, zu beseitigen und/oder zu verhindern.

16. Verwendung nach Anspruch 15, **dadurch gekennzeichnet, dass** die nicht differenzierten Zellen Zellen sind, die eine hypoproliferative Wirkung aufweisen.

17. Verwendung nach Anspruch 16, **dadurch gekennzeichnet, dass** die Zellen, die eine hypoproliferative Wirkung aufweisen, aus der Gruppe ausgewählt sind, die von den Krebszellen und den Knochenmarkszellen gebildet wird.

18. Verwendung nach Anspruch 15, **dadurch gekennzeichnet, dass** die nicht differenzierten Zellen totipotente Zellen sind.

19. Verfahren zur Neutralisierung, Beseitigung und/oder Verhinderung der Entwicklung von nicht differenzierten Zellen, die in einem flüssigen Medium vorhanden sind, in dem Gasmikrokugeln (5) einen durchschnittlichen Durchmesser von weniger als 1 mm aufweisen und Ultraschallwellen (4) in das Innere eines Fachs (2) gesendet werden, das das flüssige, zu behandelnde Medium aufweist, wobei die Gasmikrokugeln (5) in das Feld der Ultraschallwellen (4) gesendet werden, die in das Fach (2) gesendet werden, **dadurch gekennzeichnet, dass** die Ultraschallwellen (4) hochfrequente Ultraschallwellen zwischen 100 KHz und mehreren MHz inklusive sind und in dem die Gasmikrokugeln (5) Luft- oder Sauerstoff-Mikrokugeln sind.

20. Verfahren nach Anspruch 19, **dadurch gekennzeichnet, dass** das flüssige Medium ein wässriges flüssiges Medium ist.

21. Verfahren nach Anspruch 20, **dadurch gekennzeichnet, dass** das flüssige Medium ein physiologisches Medium ist, das dem Menschen oder dem Tier verabreichbar oder dem Menschen oder dem Tier entnehmbar ist.

22. Verfahren nach Anspruch 21, **dadurch gekennzeichnet, dass** die physiologische Flüssigkeit aus der Gruppe ausgewählt ist, die von dem Blut, dem Serum oder der Hirn- und Rückenmarksflüssigkeit gebildet wird.

23. Verfahren nach einem der vorangehenden Ansprüche 19 bis 22, **dadurch gekennzeichnet, dass** der durchschnittliche Durchmesser der Gasmikrokugeln (5) kleiner als 50 μm ist.

**24.** Verfahren nach einem der vorangehenden Ansprüche 19 bis 23, **dadurch gekennzeichnet, dass** der durchschnittliche Durchmesser der Gasmikrokugeln (5) kleiner als 30 $\mu$m ist.

**25.** Verfahren nach einem der vorangehenden Ansprüche 19 bis 24, **dadurch gekennzeichnet, dass** die in das Fach (2) gesendeten Ultraschallwellen (4) nicht die Erscheinung des stationären Felds erzeugen.

**26.** Verfahren nach einem der vorangehenden Ansprüche 19 bis 25, **dadurch gekennzeichnet, dass** das Licht, dessen elektromagnetische Strahlung sich mehrheitlich im Sichtbaren befindet, in das Feld der Ultraschallwellen (4) gesendet wird.

**27.** Verfahren nach einem der vorangehenden Ansprüche 19 bis 26, **dadurch gekennzeichnet, dass** die nicht differenzierten Zellen Zellen sind, die eine hypoproliferative Wirkung aufweisen.

**28.** Verfahren nach Anspruch 27, **dadurch gekennzeichnet, dass** die Zellen, die eine hypoproliferative Wirkung aufweisen, aus der Gruppe ausgewählt sind, die von den Krebszellen und den Knochenmarkszellen gebildet wird.

**29.** Verfahren nach Anspruch 27, **dadurch gekennzeichnet, dass** die Zellen, die eine hypoproliferative Wirkung aufweisen, totipotente Zellen sind.

FIG.1

FIG.2

Fig.3

FIG.4

Fig.5

EP 1 310 460 B1

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5198122 A
- DE 4407564
- JP 930343777 B
- JP 900401407 B
- JP 920035473 B
- JP 920035472 B
- JP 920035896 B
- JP 820010627 B
- US 2717874 A
- WO 9313674 A
- EP 0633049 A1
- US 4961860 A
- EP 0619104 A
- GB 1389291 A
- US 5401237 A
- EP 0515346 A
- US 4879045 A

**Littérature non-brevet citée dans la description**

- **PÉTRIER C. et al.** *Journal of Physical Chemistry, No. 98,* 1994, 10514-10520
- **UMEMURA S.I.** *Japanese J. of Cancer Res.,* 1990, vol. 81, 962-966